# EUROPÄISCHE PATENTSCHRIFT

(11) **EP 4 213 906 B1**
(45) Veröffentlichungstag und Bekanntmachung des Hinweises auf die Patenterteilung: **29.04.2026**
(21) Anmeldenummer: 21777986.7
(22) Anmeldetag: 13.09.2021
(51) Int. Cl.: A61M 1/14

(54) **MEDIZINISCHES FLUIDPUMPSYSTEM**
MEDICAL FLUID PUMP SYSTEM
SYSTÈME DE POMPE À FLUIDE MÉDICAL

(30) Priorität: 15.09.2020 DE 102020211553
(43) Veröffentlichungstag der Anmeldung: 26.07.2023
(73) Patentinhaber: B. Braun Avitum AG, 34212 Melsungen (DE)
(72) Erfinder: WOLFF, Henrik, 34212 Melsungen-Adelshausen (DE); WÜRSCHMIDT, Tobias, 34346 Hann. Münden (DE)
(74) Vertreter: Patentanwälte Ruff, Wilhelm, Beier, Dauster & Partner mbB
(86) Internationale Anmeldenummer: PCT/EP2021/075090
(87) Internationale Veröffentlichungsnummer: WO 2022/058278

(56) Entgegenhaltungen:
- US-A1- 2003 117 044
- US-A1- 2009 182 262
- US-A1- 2011 009 814
- US-A1- 2011 015 610
- US-B1- 6 905 479

## Beschreibung

Die Erfindung betrifft ein medizinisches Fluidpumpsystem zur Förderung eines Fluids bei einer extrakorporalen Blutbehandlung, aufweisend ein Gehäuse mit einem Aufnahmebereich, eine lösbar an dem Aufnahmebereich aufgenommene Fluidkassette mit einem formstabilen Grundkörper und wenigstens einer elastischen Pumpmembran, die unter Ausbildung wenigstens eines Pumpvolumens an dem Grundkörper festgelegt ist, wobei das Pumpvolumen mittels einer Auslenkung der Pumpmembran zur Förderung des Fluids veränderlich ist, und aufweisend wenigstens ein Aktorelement, das zur Auslenkung der Pumpmembran eingerichtet ist.

Ein derartiges medizinisches Fluidpumpsystem ist aus der US 2011/0015610 A1 bekannt und in Form eines Dialysegeräts zur Verwendung bei einer extrakorporalen Blutbehandlung vorgesehen. Das bekannte Dialysegerät weist eine Fluidkassette und ein Gehäuse mit einer Aufnahmeaussparung auf, die zur Aufnahme der Fluidkassette vorgesehen und mittels eines Deckels verschließbar ist. Die Fluidkassette weist einen Grundkörper und eine unter Ausbildung eines Pumpvolumens an dem Grundkörper festgelegte Pumpmembran auf. Das Pumpvolumen ist zur Pumpförderung einer Dialysierflüssigkeit vorgesehen und hierzu mittels einer entsprechenden Auslenkung der Pumpmembran volumenveränderlich. Zur Auslenkung der Pumpmembran ist eine in das Gehäuse integrierte Hubkolbenanordnung mit einem in der Aufnahmeaussparung angeordneten und hubbeweglich an dem Gehäuse geführten Hubkolben vorgesehen. In einem in der Aufnahmeaussparung eingelegten Zustand der Fluidkassette ist die Pumpmembran dem Hubkolben zugewandt, so dass eine Hubbewegung des Hubkolbens eine Auslenkung der Pumpmembran und damit die Pumpförderung der Dialysierflüssigkeit bewirkt.

Ein ähnliches Fluidpumpsystem ist aus US 2009/182262 bekannt. Aus US 2003/117044 sind Membranpumpen mit dielektrischem Elastomeraktor bekannt.

Aufgabe der Erfindung ist es, ein medizinisches Fluidpumpsystem der eingangs genannten Art bereitzustellen, das einen einfachen und robusten Aufbau aufweist.

Diese Aufgabe wird dadurch gelöst, dass das wenigstens eine Aktorelement ein dielektrischer Elastomeraktor ist, der mit der Pumpmembran wirkverbunden ist, und der unter Einwirkung einer elektrischen Spannung eine Stellbewegung zur Auslenkung der Pumpmembran ausführt. Durch die erfindungsgemäße Lösung kann insbesondere auf eine Hubkolbenanordnung zur Auslenkung der Pumpmembran verzichtet werden. Stattdessen ist erfindungsgemäß der wenigstens eine dielektrische Elastomeraktor vorgesehen. Dielektrische Elastomeraktoren, deren grundsätzlicher Aufbau sowie das zugrunde liegende Funktionsprinzip zur Wandlung von elektrischer Energie in mechanische Energie sind jedenfalls im Bereich der Antriebstechnik bekannt. Der prinzipielle Aufbau eines solchen dielektrischen Elastomeraktors weist einen passiven Elastomerfilm auf, dessen Ober- und Unterseite jeweils mit einer nachgiebigen Elektrode beschichtet ist. Wird eine elektrische Spannung an die einander gegenüberliegenden Elektroden angelegt, ziehen sich diese aufgrund elektrostatischer Kräfte an. Hierdurch wird der Elastomerfilm in Dickenrichtung zusammengedrückt und in seitlicher Richtung dementsprechend flächig ausgedehnt. In einem spannungslosen Zustand kehrt der Elastomerfilm in seinen in seitlicher Richtung zusammengezogenen und in Dickenrichtung ausgedehnten Ursprungszustand zurück. Die Stellbewegung zur Auslenkung der Pumpmembran wird auf Grundlage dieses Funktionsprinzips generiert. Der Elastomerfilm, der auch als Elastomermembran bezeichnet werden kann, ist vorzugsweise aus Silikon oder Acryl gefertigt. Die Elektroden können insbesondere aus Graphitpulver, einem Silikonöl-Graphitgemisch oder Gold gefertigt sein. Ausgehend von dem vorbeschriebenen prinzipiellen Aufbau kann der wenigstens eine dielektrische Elastomeraktor insbesondere in Form eines Planaraktors, Stapelaktors, Bündelaktors, Rollaktors oder Schalenaktors ausgebildet sein. Die vorgenannten Bauformen sind als solche grundsätzlich bekannt, so dass weitergehende diesbezügliche Erläuterungen entbehrlich sind. Durch die erfindungsgemäße Lösung wird ein konstruktiv einfacher, robuster und bauraumsparender Aufbau erreicht. Zudem hat sich gezeigt, dass die erfindungsgemäße Lösung einen vergleichsweise geräuschreduzierten Pumpbetrieb des medizinischen Fluidpumpsystems ermöglicht. Denn im Vergleich zu aus dem Stand der Technik bekannten Lösungen ermöglicht die Gestaltung des Aktorelements als dielektrischer Elastomeraktor einen weitgehenden, insbesondere vollständigen, Verzicht auf reibungs- und damit geräuschbehaftete Lager-, Führungs- oder sonstige Funktionselemente zur Lagerung oder Führung der Stellbewegung. Der Aufnahmebereich ist vorzugsweise an einer außenliegenden Gehäusewandung, insbesondere einer Gehäusefront, des Gehäuses angeordnet. Alternativ kann der Aufnahmebereich in Form eines, insbesondere mittels eines Verschlusselements verschließbaren, Aufnahmefachs, einer Aufnahmeaussparung oder dergleichen gestaltet sein. Die Fluidkassette ist relativ zu dem Gehäuse zwischen einem Aufnahmezustand und einem Entnahmezustand verlagerbar. In dem Aufnahmezustand ist die Fluidkassette an dem Aufnahmebereich des Gehäuses aufgenommen. Dementgegen ist die Fluidkassette in dem Entnahmezustand von dem Aufnahmebereich entnommen und insoweit von dem Gehäuse separiert. Vorzugsweise weist die Fluidkassette einen Fluideinlass und einen Fluidauslass auf. Bei der Förderung des Fluids gelangt dieses vorzugsweise durch den Fluideinlass in das Pumpvolumen und tritt von dort durch den Fluidauslass aus der Fluidkassette aus. Mit anderen Worten ausgedrückt, ist das Pumpvolumen vorzugsweise fluidleitend mit dem Fluideinlass und dem Fluidauslass verbunden. Der Grundkörper der Fluidkassette ist vorzugsweise aus einem formstabilen Kunststoff gefertigt. Die Pumpmembran ist elastisch, insbesondere weichelastisch, und vorzugsweise aus einem Silikonwerkstoff gefertigt. Das wenigstens eine Pumpvolumen ist abschnittsweise von der Pumpmembran und/oder abschnittsweise von dem Grundkörper umgrenzt. Die Fluidkassette kann einem extrakorporalen Blutkreislauf zugeordnet sein, insoweit als Blutkassette bezeichnet werden und zur Pumpförderung von Blut vorgesehen sein. Alternativ kann die Fluidkassette einem Dialysierflüssigkeitskreislauf zugeordnet sein, insoweit als Dialysierflüssigkeitskassette bezeichnet werden und zur Pumpförderung von Dialysierflüssigkeit vorgesehen sein. Der dielektrische Elastomeraktor ist wenigstens mittelbar mit der Pumpmembran wirkverbunden. Vorzugsweise ist der dielektrische Elastomeraktor dem Gehäuse oder der Fluidkassette zugeordnet. Im erstgenannten Fall ist der dielektrische Elastomeraktor gehäuseseitig in dem Aufnahmebereich angeordnet und insoweit stationär. Im zweitgenannten Fall ist der dielektrische Elastomeraktor kassettenseitig angeordnet und gemeinsam mit der Fluidkassette relativ zu dem Gehäuse zwischen dem Aufnahme- und dem Entnahmezustand verlagerbar. Die Stellbewegung des dielektrischen Elastomeraktors kann unmittelbar oder mittelbar auf die Pumpmembran übertragen werden. Zur unmittelbaren Übertragung können der dielektrische Elastomeraktor und die Pumpmembran kraft- und bewegungsübertragend kontaktiert sein. Zur mittelbaren Übertragung kann eine Koppeleinrichtung vorgesehen sein, die sowohl mit dem dielektrischen Elastomeraktor als auch mit der Pumpmembran wirkverbunden ist, so dass die Kraft- und Bewegungsübertragung von dem dielektrischen Elastomeraktor über die Koppeleinrichtung auf die Pumpmembran erfolgt.

In Ausgestaltung der Erfindung ist der wenigstens eine dielektrische Elastomeraktor in das Gehäuse integriert. Dies gestattet insbesondere einen besonders einfachen Aufbau der Fluidkassette. Hierdurch kann diese besonders kostengünstig, insbesondere als Einwegartikel zur einmaligen Verwendung, hergestellt werden. Bei dieser Ausgestaltung der Erfindung ist die Pumpmembran vorzugsweise außenliegend an dem Grundkörper festgelegt, so dass eine dem Pumpvolumen abgewandte Außenseite der Pumpmembran dem Aufnahmebereich zugewandt ist. Weiter vorzugsweise ist der dielektrische Elastomeraktor wenigstens abschnittsweise außenliegend an dem Aufnahmebereich angeordnet und hierdurch wenigstens mittelbar, vorzugsweise unmittelbar, mit der außenliegenden Pumpmembran kontaktierbar.

In weiterer Ausgestaltung der Erfindung ist der wenigstens eine dielektrische Elastomeraktor ein flächig in dem Aufnahmebereich angeordneter Planaraktor. Der Planaraktor kann auch als dielektrischer Membranaktor oder dielektrische Aktormembran bezeichnet werden und weist eine flächige Ausdehnung mit einer senkrecht zu dieser Ausdehnung orientierten Stellbewegung auf. Hierdurch kann der Planaraktor in besonders bauraumsparender Weise in das Gehäuse integriert werden.

In weiterer Ausgestaltung der Erfindung ist eine an dem Gehäuse und/oder der Fluidkassette angeordnete Koppeleinrichtung vorgesehen, mittels derer der wenigstens eine dielektrische Elastomeraktor und die Pumpmembran - in einem an dem Aufnahmebereich aufgenommenen Zustand der Fluidkassette - kraft- und/oder bewegungsübertragend miteinander gekoppelt sind. Die Koppeleinrichtung dient insbesondere einer mittelbaren Übertragung der Stellbewegung und der aus dieser resultierenden Stellkräfte des dielektrischen Elastomeraktors auf die Pumpmembran. Hierdurch können der dielektrische Elastomeraktor und die Pumpmembran - im Aufnahmezustand der Fluidkassette - voneinander beabstandet und insbesondere im Inneren des Gehäuses und/oder des Grundkörpers angeordnet sein. Eine solche innenliegende Anordnung beugt insbesondere einer Beschädigung des dielektrischen Elastomeraktors und/oder der Pumpmembran vor. Die Koppeleinrichtung bewirkt vorzugsweise eine mechanische Kopplung. Alternativ oder zusätzlich kann eine magnetische Kopplung bewirkt sein.

In weiterer Ausgestaltung der Erfindung weist die Koppeleinrichtung eine Koppelfeder auf, die einends an dem dielektrischen Elastomeraktor und andernends an der Pumpmembran abgestützt ist. Die Koppelfeder bewirkt eine in Richtung der Stellbewegung des dielektrischen Elastomeraktors und/oder in Richtung der Auslenkung der Pumpmembran elastisch nachgiebige Kopplung. Dies dient insbesondere einem Toleranzausgleich, so dass die Stellbewegung möglichst spielfrei in eine Auslenkung der Pumpmembran übertragen wird und insbesondere ein Leerhub des dielektrischen Elastomeraktors vermieden wird. Die Koppelfeder kann auch als Toleranzfeder bezeichnet werden. Die Koppelfeder ist vorzugsweise als Wendel-, Blatt- oder Scheibenfeder gestaltet.

In weiterer Ausgestaltung der Erfindung weist die Fluidkassette mehrere elastische Pumpmembranen auf, die unter Ausbildung jeweils eines Pumpvolumens an dem Grundkörper festgelegt sind, und es sind mehrere dielektrische Elastomeraktoren vorgesehen, die mit jeweils einer der mehreren Pumpmembranen wirkverbunden sind. Eine solche Gestaltung mit mehreren Pumpmembranen und mehreren dielektrischen Elastomeraktoren gestattet insbesondere eine alternierende Pumpförderung, bei welcher die mehreren Pumpvolumen zeitversetzt, nacheinander und/oder abwechselnd entleert und befüllt werden. Hierdurch kann eine Verstetigung der Pumpförderung erreicht werden.

In weiterer Ausgestaltung der Erfindung ist der wenigstens eine dielektrische Elastomeraktor in die Fluidkassette integriert und gemeinsam mit dieser lösbar an dem Aufnahmebereich aufgenommen. Hierdurch kann das Gehäuse des Fluidpumpsystems besonders einfach, kompakt und kostengünstig aufgebaut werden. Gleichzeitig ermöglicht die Gestaltung des Aktorelements als dielektrischer Elastomeraktor einen vergleichsweise einfachen, kompakten und kostengünstigen Aufbau der Fluidkassette. Mit anderen Worten ausgedrückt, wird eine solche Integration durch die Gestaltung des Aktorelements als dielektrischer Elastomeraktor überhaupt erst technisch praktikabel und wirtschaftlich sinnvoll. Hierzu im Unterschied verbietet sich eine entsprechende Integration beispielsweise einer Hubkolbenanordnung aus konstruktiven und/oder wirtschaftlichen Gründen. Zur Versorgung des wenigstens einen dielektrischen Elastomeraktors weist die Fluidkassette vorzugsweise wenigstens ein elektrisches Kontaktelement auf, das - im Aufnahmezustand der Fluidkassette - mit einem komplementären Kontaktelement des Gehäuses elektrisch kontaktiert ist. Durch eine solche Kontaktierung kann in vorteilafter Weise auf eine gesonderte elektrische Steckverbindung verzichtet und stattdessen eine gleichsam automatische elektrische Verbindung beim Anbringen der Fluidkassette an dem Aufnahmebereich ausgebildet werden.

In weiterer Ausgestaltung der Erfindung ist der wenigstens eine dielektrische Elastomeraktor als Planaraktor gestaltet, wobei der Planaraktor in die Pumpmembran integriert ist oder umgekehrt. Hierdurch kann ein nochmals vereinfachter und besonders kompakter Aufbau erreicht werden. Der Planaraktor weist vorzugsweise einen Elastomerfilm oder eine Elastomerschicht auf, deren Ober- und Unterseite jeweils mit einer nachgiebigen Elektrode beschichtet ist. Vorzugsweise sind wenigstens der Elastomerfilm bzw. die Elastomerschicht und die Elektroden in die Pumpmembran eingebettet und/oder bilden gemeinsam mit der Pumpmembran eine Membrananordnung.

In weiterer Ausgestaltung der Erfindung ist die Pumpmembran unter Ausbildung eines ersten Pumpvolumens und eines zweiten Pumpvolumens an dem Grundkörper festgelegt, wobei das erste Pumpvolumen und das zweite Pumpvolumen mittels der Pumpmembran fluiddicht voneinander getrennt und wechselweise veränderlich sind. Je nach Richtung der Auslenkung der Pumpmembran wird das erste Pumpvolumen verringert und das zweite Pumpvolumen vergrößert oder umgekehrt. Dieser Aufbau ermöglicht in einfacher Weise eine verstetigte Pumpförderung des Fluids. Gleichzeitig kann im Vergleich zu einer Gestaltung, bei welcher zur Ausbildung zweier Pumpvolumen zwei separate Pumpmembranen an dem Grundkörper festgelegt sind, Bauraum eingespart werden. Diese Ausgestaltung der Erfindung ist besonders vorteilhaft in Kombination mit einer Gestaltung des dielektrischen Elastomeraktors als in die Pumpmembran integrierter Planaraktor.

In weiterer Ausgestaltung der Erfindung ist der wenigstens eine dielektrische Elastomeraktor zur kapazitiven Erfassung einer Stellposition der Stellbewegung eingerichtet und fungiert somit zusätzlich als Sensorelement. Die unter Einwirkung der elektrischen Spannung ausgeführte Stellbewegung wird durch die bereits beschriebene Geometrieänderung des dielektrischen Elastomeraktors bewirkt. Diese Geometrieänderung bewirkt gleichzeitig eine Kapazitätsänderung, die für sensorische Zwecke, beispielsweise zur Weg- und/oder Druckmessung, genutzt werden kann. Dem wenigstens einen dielektrischen Elastomeraktor kommt demnach bei dieser Ausgestaltung der Erfindung eine besonders vorteilhafte Mehrfachfunktion zu. Infolgedessen kann insbesondere auf ein gesondertes Sensorelement zur Erfassung der Stellposition verzichtet werden, wodurch ein nochmals vereinfachter Aufbau erreicht werden kann. Durch die Erfassung der Stellposition ist mittelbar auch eine Auslenkungsposition der Pumpmembran und hierdurch letztlich auch ein in dem Pumpvolumen vorherrschender Fluiddruck ermittelbar.

In weiterer Ausgestaltung der Erfindung ist eine mit der Pumpmembran wirkverbundene Notantriebseinrichtung vorgesehen, welche ein zur manuell angetriebenen Auslenkung der Pumpmembran betätigbares Antriebselement aufweist. Die manuelle Betätigung des Antriebselements bewirkt eine Stellbewegung der Notantriebseinrichtung zur Auslenkung der Pumpmembran. Die Notantriebseinrichtung gewährleistet einen Notbetrieb des medizinischen Fluidpumpsystems, bei welchem auch bei einem Versagen des dielektrischen Elastomeraktors und/oder bei einer Unterbrechung der elektrischen Spannungsversorgung die Förderung des Fluids aufrechterhalten werden kann. Das Antriebselement kann insbesondere als Kurbel, Handrad oder dergleichen gestaltet sein.

In weiterer Ausgestaltung der Erfindung ist eine Erfassungseinrichtung vorgesehen, welche zur Erfassung eines Aufnahmezustands der Fluidkassette eingerichtet ist, in welchem die Fluidkassette bestimmungsgemäß an dem Aufnahmebereich aufgenommen ist. Zu diesem Zweck kann die Erfassungseinrichtung wenigstens ein Sensorelement und/oder eine zwischen dem Aufnahmebereich und der Fluidkassette ausgebildete elektrische Kontakteinrichtung aufweisen, die im Aufnahmezustand elektrisch leitend kontaktiert und im Entnahmezustand elektrisch getrennt ist. Das Erfassen des Aufnahmezustands beugt insbesondere einer Fehlbedienung des medizinischen Fluidpumpsystems vor und erhöht die Patientensicherheit.

In weiterer Ausgestaltung der Erfindung ist der Aufnahmebereich an einer außenliegenden Gehäusewandung des Gehäuses angeordnet, und es ist eine an der Gehäusewandung und/oder dem Grundkörper ausgebildete Befestigungseinrichtung vorgesehen, mittels derer die Fluidkassette - in einem an dem Aufnahmebereich aufgenommenen Zustand - an der Gehäusewandung lösbar befestigt ist. Die Befestigungseinrichtung ist vorzugsweise als Rasteinrichtung mit mehreren Rastelementen oder als Clipseinrichtung mit mehreren Clipsen ausgebildet. Durch die außenliegende Anordnung des Aufnahmebereichs kann die Fluidkassette besonders einfach an dem Gehäuse angebracht und von diesem entnommen werden. Die Befestigungseinrichtung wirkt hierbei einem ungewollten Lösen der Fluidkassette entgegen.

In weiterer Ausgestaltung der Erfindung ist die Fluidkassette als Einwegartikel zur einmaligen Verwendung gestaltet. Hierdurch können eine aufwändige Sterilisierung der Fluidkassette vor einer nochmaligen Verwendung vermieden und hierfür anfallende Kosten eingespart werden. Zudem bietet die Gestaltung als Einwegartikel hygienische Vorteile gegenüber einer Sterilisierung und Wiederverwendung der Fluidkassette.

Die Erfindung betrifft zudem eine medizinische Fluidkassette für ein medizinisches Fluidpumpsystem gemäß der vorstehenden Beschreibung, aufweisend einen formstabilen Grundkörper und wenigstens eine elastische Pumpmembran, die unter Ausbildung wenigstens eines Pumpvolumens an dem Grundkörper festgelegt ist, wobei das Pumpvolumen mittels einer Auslenkung der Pumpmembran zur Förderung des Fluids veränderlich ist, wobei ein mit der Pumpmembran wirkverbundener dielektrischer Elastomeraktor vorgesehen ist, der unter Einwirkung einer elektrischen Spannung eine Stellbewegung zur Auslenkung der Pumpmembran ausführt. Der wenigstens eine dielektrische Elastomeraktor ist in die Fluidkassette integriert. Zur Vermeidung von Wiederholungen wird auf die Beschreibung des medizinischen Fluidpumpsystems nach Anspruch 7 verwiesen. Das zu der dortigen Fluidkassette Gesagte gilt vorzugsweise sinngemäß für die erfindungsgemäße medizinische Fluidkassette. Entsprechendes gilt für Ausgestaltungen der erfindungsgemäßen medizinischen Fluidkassette.

Weitere Vorteile und Merkmale der Erfindung ergeben sich aus den Ansprüchen sowie aus der nachfolgenden Beschreibung bevorzugter Ausführungsbeispiele der Erfindung, die anhand der Zeichnungen dargestellt sind.
- Fig. 1: zeigt eine schematisch stark vereinfachte Darstellung eines Ausschnitts eines medizinischen Geräts mit einer Ausgestaltung eines erfindungsgemäßen medizinischen Fluidpumpsystems mit einem Gehäuse und einer lösbar an einem Aufnahmebereich des Gehäuses aufgenommenen Fluidkassette,
- Fig. 2: in schematisch stark vereinfachter Detaildarstellung den mit zwei Aktorelementen versehenen Aufnahmebereich des Gehäuses in einer Frontansicht, wobei die Fluidkassette von dem Aufnahmebereich entnommen ist,
- Fig. 3: eine weitere Detaildarstellung des Aufnahmebereichs mit der an diesem aufgenommenen Fluidkassette in einer schematisch stark vereinfachten Schnittdarstellung entlang einer Schnittlinie III-III nach Fig. 2,
- Fig. 4: eine vereinfachte Detaildarstellung der Fluidkassette entlang einer Schnittlinie IV-IV nach Fig. 3, wobei die Fluidkassette zwei Pumpmembranen aufweist,
- Fig. 5: eine weitere Detaildarstellung der Fluidkassette entlang einer Schnittlinie V-V nach Fig. 4,
- Fig. 6, 7: schematisch stark vereinfachte Prinzipdarstellungen der anhand Fig. 2 gezeigten Aktorelemente in unterschiedlichen Zuständen,
- Fig. 8: eine weitere vergrößerte Prinzipdarstellung des Fluidpumpsystems im Bereich eines der Aktorelemente,
- Fig. 9: eine schematisch stark vereinfachte Prinzipdarstellung im Bereich einer Koppeleinrichtung und
- Fig. 10: eine Detaildarstellung einer weiteren Ausführungsform eines erfindungsgemäßen medizinischen Fluidpumpsystems.

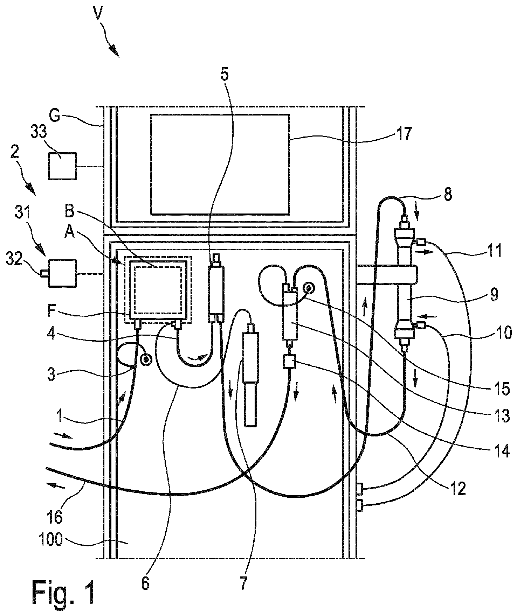

Gemäß Fig. 1 ist ein medizinisches Gerät V zur extrakorporalen Blutbehandlung abschnittsweise gezeigt und in Form eines Dialysegeräts gestaltet. Fig. 1 zeigt in schematisch stark vereinfachter Weise im Wesentlichen einen gesamten extrakorporalen Blutkreislauf des medizinischen Geräts V. Dieser weist eine arterielle Blutleitung 1 auf, mittels derer zu behandelndes Blut von einem nicht gezeigten Patienten zu einem Fluidpumpsystem 2 des medizinischen Geräts V geführt ist. In Bezug auf eine Förderrichtung des Bluts vor dem Fluidpumpsystem 2 ist ein arterieller Druckabnehmer 3 vorgesehen. Mittels des arteriellen Druckabnehmers 3 ist der Druck in der arteriellen Blutleitung 1 vor dem Fluidpumpsystem 2 erfassbar. Dieser Druck kann auch als niederdruckseitiger Druck bezeichnet werden. In Förderrichtung des Bluts nach dem Fluidpumpsystem 2 - und somit hochdruckseitig - führt eine Hochdruckblutleitung 4 zu einer arteriellen Luftfalle 5. An einem Auslass des Fluidpumpsystems 2 ist vorliegend eine Zuleitung 6 angeordnet, welche an einer Pumpe 7 angeschlossen ist. Über die Zuleitung 6 kann Zusatzstoff, beispielsweise Heparin zur Blutverdünnung, hinzudosiert werden. Ausgehend von der arteriellen Luftfalle 5 führt eine Leitung 8 das zu behandelnde Blut zu einem Dialysator 9, welchem eingangsseitig Dialysierflüssigkeit über eine Dialysierflüssigkeitszuleitung 10 zugeführt ist. In dem Dialysator 9 wird das Blut in einer bekannten Weise mittels der Dialysierflüssigkeit behandelt. Verbrauchte Dialysierflüssigkeit, die auch als Dialysat bezeichnet werden kann, wird über eine Dialysierflüssigkeitsableitung 11 von dem Dialysator 9 abgeleitet und einer nicht dargestellten Entsorgung oder Aufbereitung zugeführt. Das behandelte Blut wird mittels einer Blutableitung 12 von dem Dialysator 9 zu einer venösen Luftfalle 13 zur Abscheidung von Luft geleitet. Dieser nachgeschaltet ist ein Luftdetektor 14, der erkennt, ob sich für den Patienten gefährliche Luft in dem System befindet. An der venösen Luftfalle 13 ist ein venöser Druckabnehmer 15 vorgesehen, mittels dessen der venöse Druck erfassbar ist. Von der venösen Luftfalle 13 über den Luftdetektor 14 wird das behandelte Blut über eine venöse Blutleitung 16 zurück zu dem Patienten geleitet. Zudem ist eine Steuer- und Überwachungseinrichtung 17 zur Steuerung und Überwachung des medizinischen Geräts V vorgesehen.

Das Fluidpumpsystem 2 dient der Pumpförderung des Bluts durch den vorbeschriebenen extrakorporalen Blutkreislauf des medizinischen Geräts V und weist ein Gehäuse G und eine lösbar an dem Gehäuse G aufgenommene Fluidkassette F auf.

Bei der gezeigten Ausführungsform ist das Fluidpumpsystem 2 insoweit in das medizinische Gerät V integriert, als das Gehäuse G gleichzeitig dem medizinischen Gerät V zugeordnet ist. Bei einer nicht gezeigten Ausführungsform weist das Fluidpumpsystem stattdessen ein separates Gehäuse auf, so dass das Fluidpumpsystem als separate, aber dem medizinischen Gerät zugeordnete Funktionseinheit ausgebildet ist.

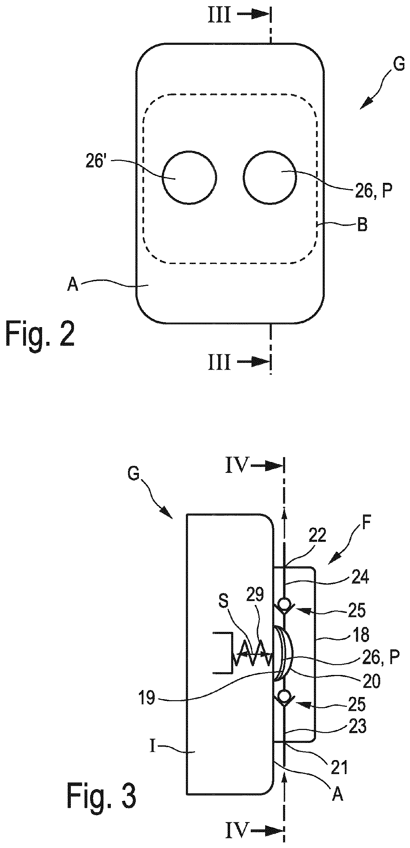

Das Gehäuse G weist einen Aufnahmebereich A auf (Fig. 2), der zur lösbaren Aufnahme der Fluidkassette F vorgesehen ist. Der Aufnahmebereich A ist bei der gezeigten Ausführungsform an einer Gehäusefront 100 des Gehäuses G angeordnet.

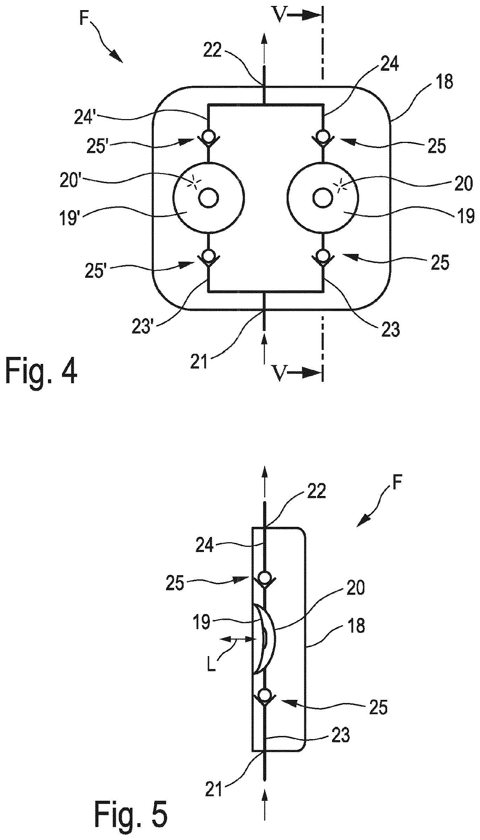

Die Fluidkassette F ist anhand der Fig. 1 und 3 in einem an dem Aufnahmebereich A aufgenommenen Aufnahmezustand gezeigt und weist einen formstabilen Grundkörper 18 und wenigstens eine elastische Pumpmembran 19 auf, die unter Ausbildung wenigstens eines Pumpvolumens 20 an dem Grundkörper 18 festgelegt ist (Fig. 4, 5). Das Pumpvolumen 20 bildet einen fluidführenden Abschnitt des extrakorporalen Blutkreislaufs. Zu diesem Zweck ist das Pumpvolumen 20 einerseits fluidleitend mit einem Fluideinlass 21 und andererseits fluidleitend mit einem Fluidauslass 22 der Fluidkassette F verbunden. Hierfür weist die Fluidkassette F eine zwischen dem Fluideinlass 21 und dem Pumpvolumen 20 erstreckte Fluidzuleitung 23 und eine zwischen dem Pumpvolumen 20 und dem Fluidauslass 22 erstreckte Fluidableitung 24 auf. Der Fluidzuleitung 23 und der Fluidableitung 24 ist jeweils ein Rückschlagventil 25 zugeordnet.

Die Pumpförderung des Bluts erfolgt unter Bezugnahme auf Fig. 5 prinzipiell folgendermaßen: Zum Ansaugen von Blut durch den Fluideinlass 21 wird die Pumpmembran 19 nach links - und damit in Richtung einer Außenseite des Grundkörpers 18 - ausgelenkt. Hierdurch wird das Pumpvolumen 20 vergrößert. Der hierbei entstehende Unterdruck öffnet das der Fluidzuleitung 23 zugeordnete Rückschlagventil 25, so dass Blut durch den Fluideinlass 21 über die Fluidzuleitung 23 in das Pumpvolumen 20 angesaugt wird. Das der Fluidableitung 24 zugeordnete Rückschlagventil 25 bleibt hierbei geschlossen. Zum Ausstoßen des zuvor angesaugten Bluts aus dem Pumpvolumen 20 wird die Pumpmembran 19 nach rechts - und damit in Richtung einer Innenseite des Grundkörpers 18 - ausgelenkt. Hierdurch wird das Pumpvolumen 20 verringert. Der damit einhergehende Überdruck öffnet das der Fluidableitung 24 zugeordnete Rückschlagventil 25, so dass das zuvor angesaugte Blut über die Fluidableitung 24 aus dem Pumpvolumen 20 durch den Fluidauslass 22 gefördert wird. Das der Fluidzuleitung 23 zugeordnete Rückschlagventil 25 bleibt hierbei geschlossen.

Zur Auslenkung der Pumpmembran 19 weist das Fluidpumpsystem 2 wenigstens ein Aktorelement in Form eines dielektrischen Elastomeraktors 26 auf (Fig. 2, 3). Bei der Ausführungsform nach den Fig. 1 bis 9 ist der dielektrische Elastomeraktor 26 in das Gehäuse G integriert. Der dielektrische Elastomeraktor 26 ist in dem insbesondere anhand der Fig. 1 und 3 gezeigten Aufnahmezustand der Fluidkassette F mit der Pumpmembran 19 wirkverbunden und führt unter Einwirkung einer elektrischen Spannung eine Stellbewegung S zur Auslenkung der Pumpmembran 19 aus (Fig. 3).

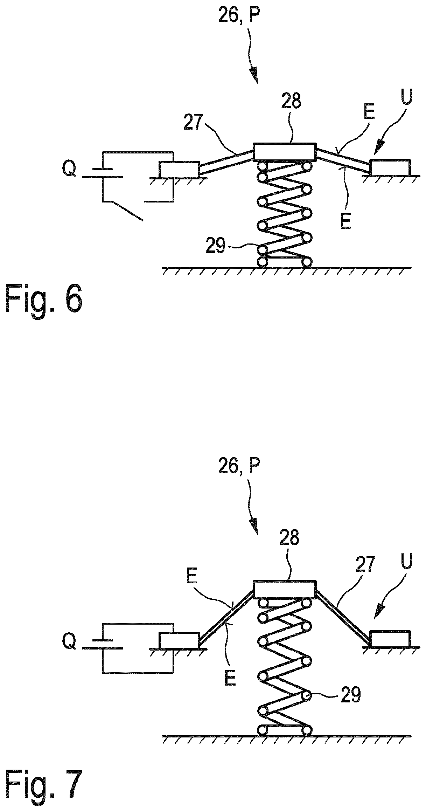

Anhand der Fig. 6 und 7 ist eine mögliche Ausgestaltung des dielektrischen Elastomeraktors 26 gezeigt. Die Fig. 6 und 7 sind als schematisch stark vereinfachte Prinzipdarstellungen zur Verdeutlichung des Funktionsprinzips und des grundsätzlichen Aufbaus des dielektrischen Elastomeraktors 26 zu verstehen. Bei der gezeigten Ausgestaltung weist der dielektrische Elastomeraktor 26 eine passive Elastomermembran 27 auf, welche auch als Elastomerfilm bezeichnet werden kann. Die Elastomermembran 27 ist vorliegend kreisscheibenförmig gestaltet und an ihrem Außenumfang U an nicht näher bezeichneten Lagerstellen gehäuseseitig fest gelagert. Weiter weist der dielektrische Elastomeraktor 26 ein Stützelement 28 auf, das auf eine dem Fachmann bekannte Weise mit einem Innenumfang der Elastomermembran 27 gekoppelt ist. Das Stützelement 28 ist vorliegend als kreisförmige Platte gestaltet. An einer nicht näher bezeichneten Unterseite des Stützelements 28 greift eine Stellfeder 29 an. Andernends ist die Stellfeder 29 gehäuseseitig abgestützt. An einer Oberseite und an einer Unterseite der Elastomermembran 27 ist jeweils eine Elektrode E angeordnet. Die Elektroden E sind auf eine dem Fachmann bekannte Weise ausgebildet und an eine Spannungsquelle Q angeschlossen. In dem anhand Fig. 7 gezeigten Zustand ist eine elektrische Spannung an die Elektroden E angelegt. Diese ziehen sich somit aufgrund elektrostatischer Kräfte an. Hierdurch wird die Elastomermembran 27 in ihrer Dickenrichtung komprimiert, was zu einer flächigen Ausdehnung in radialer Richtung führt. Ausgehend von dem anhand Fig. 6 gezeigten spannungslosen Zustand wird die Elastomermembran 27 infolge der flächigen Ausdehnung und des damit einhergehenden mechanischen Vorspannungsverlusts durch die Stellfeder 29 ausgelenkt. Wird die elektrische Verbindung zu der Spannungsquelle Q getrennt (Fig. 6), kehrt die Elastomermembran 27 in ihren in radialer Richtung zusammengezogenen und in Dickenrichtung ausgedehnten Zustand zurück. Dabei kompensiert die radiale mechanische Vorspannung der Elastomermembran 27 die Federkraft der Stellfeder 29. Die Stellbewegung S des dielektrischen Elastomeraktors 26 wird auf Grundlage des vorbeschriebenen Funktionsprinzips generiert, das als solches bekannt ist, so dass von weiteren diesbezüglichen Erläuterungen abgesehen werden kann.

Bei der gezeigten Ausführungsform ist der dielektrische Elastomeraktor 26 als flächig in dem Aufnahmebereich A angeordneter Planaraktor P gestaltet. Bei zeichnerisch nicht dargestellten Ausführungsformen ist der dielektrische Elastomeraktor stattdessen als Stapelaktor, Bündelaktor, Rollaktor oder Schalenaktor ausgebildet. Die vorgenannten Bauformen dielektrischer Elastomeraktoren sind als solche bekannt, so dass weitergehend diesbezügliche Erläuterungen entbehrlich sind.

Bei einer zeichnerisch nicht dargestellten Ausführungsform sind mehrere in Förderrichtung hintereinandergeschaltete dielektrische Elastomeraktoren vorgesehen, die nach Art einer linearen Peristaltik mit der Pumpmembran gekoppelt sind. In diesem Fall ist die Pumpmembran unter Ausbildung eines entlang der Förderrichtung längserstreckten Pumpvolumens an dem Grundkörper festgelegt. Zur Pumpförderung des Bluts führen die in Reihe angeordneten Elastomeraktoren gleichsam wellenförmige Stellbewegungen aus, wodurch das Blut entlang des Pumpvolumens gefördert wird. Bei einer solchen Ausgestaltung kann insbesondere auf Rückschlagventile verzichtet und ein nochmals vereinfachter Aufbau erreicht werden.

Anhand Fig. 3 ist der Planaraktor P in einer oberen Endlage der Stellbewegung S dargestellt (vgl. Fig. 7). In diesem Zustand ist die Elastomermembran 27 in Bezug auf die Gehäusefront 100 konvex aus dem Aufnahmebereich A herausgewölbt. Dementgegen ist die Elastomermembran 27 in der unteren Endlage (Fig. 6) flächenbündig mit der Gehäusefront 100 und/oder dem Aufnahmebereich A.

Bei der anhand der Fig. 1 bis 9 gezeigten Ausführungsform wirkt der Planaraktor P - im Aufnahmezustand der Fluidkassette F - unmittelbar mechanisch auf die Pumpmembran 19 ein. Mit anderen Worten ausgedrückt, kontaktiert eine dem Pumpvolumen 20 abgewandte Außenseite der Pumpmembran 19 eine einem Innenraum I des Gehäuses G abgewandte Außenseite des Planaraktors P.

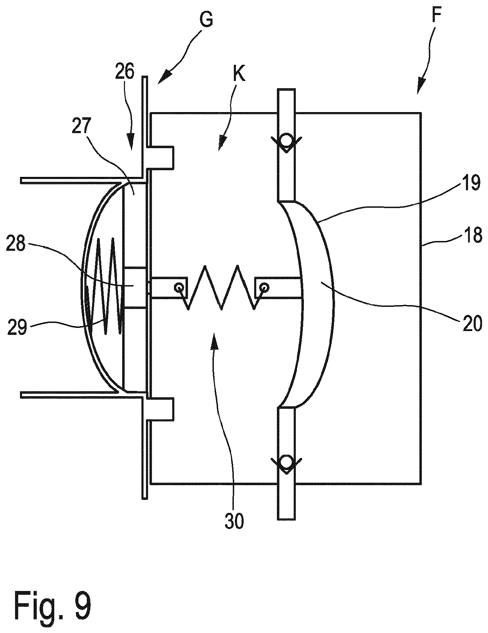

Um eine toleranzgerechte Ankopplung des dielektrischen Elastomeraktors 26 an die Pumpmembran 19 zu gewährleisten, ist vorzugsweise eine Koppeleinrichtung vorgesehen. Eine mögliche Ausgestaltung einer Koppeleinrichtung ist schematisch vereinfacht insbesondere anhand Fig. 9 gezeigt. Die Koppeleinrichtung K weist vorliegend eine Koppelfeder 30 auf, die einends an dem dielektrischen Elastomeraktor 26 und andernends an der Pumpmembran 19 abgestützt ist. Die Koppelfeder 30 ist nicht mit der Stellfeder 29 des dielektrischen Elastomeraktors 26 zu verwechseln (Fig. 6, 7). Die Koppelfeder 30 bewirkt eine entlang der Stellbewegung S und damit entlang der Auslenkung L orientierte Vorspannung zwischen dem dielektrischen Elastomeraktor 26, genauer: dessen Elastomermembran 27, und der Pumpmembran 19. Diese Vorspannung wirkt spielreduzierend. Die beiderseitige Abstützung der Koppelfeder 30 ist zug- und druckkraftübertragend gestaltet. Die Koppeleinrichtung K nach Fig. 9 ist der Fluidkassette F zugeordnet. Bei einer nicht gezeigten Ausführungsform ist eine dem Gehäuse zugeordnete Koppeleinrichtung vorgesehen.

Wie weiter anhand Fig. 4 gezeigt ist, weist die Fluidkassette F vorliegend mehrere Pumpmembranen 19, 19' und dementsprechend mehrere Pumpvolumen 20, 20' auf. Dabei sind bei der gezeigten Ausführungsform genau zwei Pumpmembranen 19, 19' und dementsprechend zwei Pumpvolumen 20, 20' vorgesehen, die auch als erste Pumpmembran 19, erstes Pumpvolumen 20, zweite Pumpmembran 19' und zweites Pumpvolumen 20' bezeichnet werden können. Bei nicht gezeigten Ausführungsformen sind drei, vier, fünf und/oder mehr Pumpmembranen und damit auch Pumpvolumen vorgesehen. Die zweite Pumpmembran 19' ist unter Ausbildung des zweiten Pumpvolumens 20' an dem Grundkörper 18 festgelegt. Das zweite Pumpvolumen 20' ist in einer dem ersten Pumpvolumen 20 entsprechenden Weise mittels einer Fluidzuleitung 23' und einer Fluidableitung 24' mit dem Fluideinlass 21 bzw. dem Fluidauslass 22 der Fluidkassette F fluidleitend verbunden. Zur Steuerung des Fluid- bzw. Blutflusses sind wiederum Rückschlagventile 25' vorgesehen und der Fluidzuleitung 23' und der Fluidableitung 24' zugeordnet.

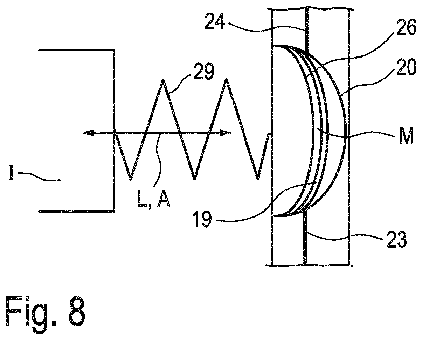

Gehäuseseitig weist das Fluidpumpsystem 2 dementsprechend mehrere dielektrische Elastomeraktoren 26, 26' auf, die auch als erster Elastomeraktor 26 und zweiter Elastomeraktor 26' bezeichnet werden können. Der zweite Elastomeraktor 26' ist baugleich zu dem ersten Elastomeraktor 26 und insoweit ebenfalls als Planaraktor gestaltet. Die beiden Elastomeraktoren 26, 26' sind in einer maßlich auf die Anordnung der Pumpmembranen 19, 19' an der Fluidkassette F abgestimmten Weise in dem Aufnahmebereich A angeordnet. Der zweite Elastomeraktor 26' ist in Entsprechung zu dem ersten Elastomeraktor 26 mittels einer weiteren nicht ersichtlichen Koppeleinrichtung, wie in Fig. 8 oder alternativ Fig. 9 gezeigt, an die zweite Pumpmembran 19' gekoppelt.

Die vorbeschriebene Ausgestaltung mit mehreren Pumpvolumen 20, 20' ist insbesondere im Hinblick auf eine Verstetigung der Pumpförderung und die Vermeidung von Druckspitzen vorteilhaft. Hierzu können die Elastomeraktoren 26, 26' alternierend zur abwechselnden Auslenkung der beiden Pumpmembranen 19, 19' angesteuert werden. Eine solche Gestaltung ist jedoch nicht zwingend. Bei einer nicht gezeigten Ausführungsform weist das Fluidpumpsystem genau ein Pumpvolumen und genau einen dielektrischen Elastomeraktor auf.

Zur Befestigung der Fluidkassette F an dem Gehäuse G ist eine Befestigungseinrichtung B vorgesehen, die anhand der Fig. 1 und 2 schematisch stark vereinfacht dargestellt ist. Die Befestigungseinrichtung B ist vorzugsweise als Rast- oder Clipseinrichtung gestaltet und gestattet eine werkzeuglose lösbare Befestigung der Fluidkassette F an dem Gehäuse G, genauer der Gehäusefront 100. Die Befestigungseinrichtung B weist vorzugsweise gehäuseseitige Befestigungsabschnitte oder -elemente und hierzu komplementäre kassettenseitige Befestigungsabschnitte oder -elemente auf, die (ohne Bezugszeichen) in Fig. 9 schematisch gezeigt sind.

Bei der gezeigten Ausführungsform weist das Fluidpumpsystem 2 zudem eine Notantriebseinrichtung 31 mit einem manuell betätigbaren Antriebselement 32 auf. Die Notantriebseinrichtung 31 ist schematisch stark vereinfacht anhand Fig. 1 gezeigt, wobei die strichlierte Verbindung mit dem Gehäuse G eine Wirkverbindung mit den Pumpmembranen 19, 19' symbolisiert. Das Antriebselement 32 kann beispielsweise als Kurbel oder Handrad gestaltet sein. Mittels der Notantriebseinrichtung 31 kann die Pumpförderung auch dann aufrechterhalten werden, wenn die Spannungsquelle Q (Fig. 6, 7) ausfallen oder die Elastomeraktoren 26, 26' anderweitig in ihrer Funktion beeinträchtigt sein sollten.

Weiterhin weist das Fluidpumpsystem 2 vorliegend eine Erfassungseinrichtung 33 auf, die zur Erfassung einer bestimmungsgemäßen Aufnahme und/oder Befestigung der Fluidkassette F an dem Aufnahmebereich A eingerichtet ist. Die Erfassungseinrichtung 33 kann hierfür gehäuse- und/oder kassettenseitige elektrische Kontaktelemente, Sensoren oder dergleichen aufweisen. Die Erfassungseinrichtung 33 ist vorliegend signaltechnisch mit der Steuer- und Überwachungseinrichtung 17 verbunden. Sobald die Erfassungseinrichtung 33 eine bestimmungsgemäße Aufnahme der Fluidkassette F erfasst, wird ein dementsprechendes Signal an die Steuer- und Überwachungseinrichtung 17 übermittelt und eine Inbetriebnahme des medizinischen Geräts V freigegeben. Bei Ausbleiben dieses Signals kann die Inbetriebnahme steuerungstechnisch blockiert sein. Alternativ oder zusätzlich kann ein optisch oder akustisch wahrnehmbares Warnsignal an eine Bedienperson des medizinischen Geräts V ausgegeben werden.

Bei der gezeigten Ausführungsform fungieren die dielektrischen Elastomeraktoren 26, 26' gleichzeitig als nicht näher bezeichnete Sensorelemente zur kapazitiven Erfassung einer Stellposition der jeweiligen Stellbewegung. Die anhand der Fig. 6 und 7 verdeutlichte Geometrieänderung der Elastomermembran 27 bewirkt gleichzeitig eine Kapazitätsänderung, die für sensorische Zwecke, beispielsweise für die besagte Positionsermittlung, genutzt werden kann. Die kapazitive Erfassung der Stellposition erlaubt einen Rückschluss auf eine Position der Auslenkung L der jeweiligen Pumpmembran 19, 19'. Ausgehend von der Auslenkungsposition kann auf der Grundlage grundsätzlich bekannter physikalischer Zusammenhänge auf einen in dem jeweiligen Pumpvolumen 20, 20' vorherrschenden Fluiddruck geschlossen werden. Dies ist besonders vorteilhaft, da auf für diesen Zweck vorgesehene gesonderte Sensorelemente verzichtet werden kann. Eine solche gleichzeitige Nutzung als Aktor einerseits und Sensor andererseits wird auch als "self-sensing" bezeichnet.

Wie weiter anhand Fig. 8 angedeutet ist, kann der dielektrische Elastomeraktor 26 alternativ oder zusätzlich mit einer weiteren dielektrischen Elastomermembran M versehen sein, die auf Grundlage der vorbeschriebenen Prinzipien als Sensorelement fungiert.

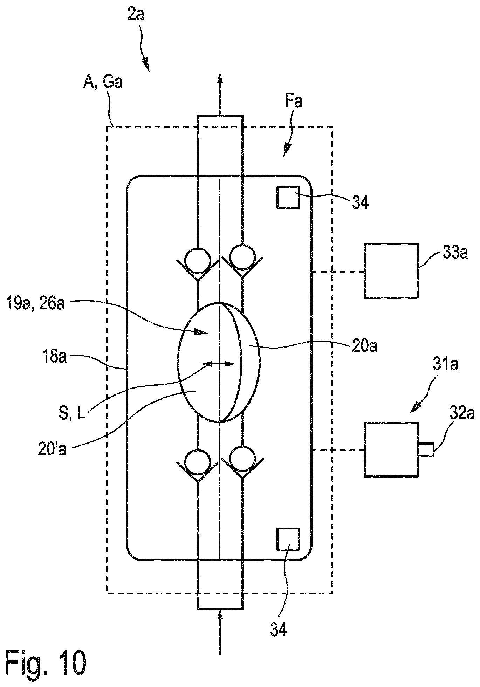

Anhand Fig. 10 ist eine weitere Ausführungsform eines erfindungsgemäßen Fluidpumpsystems 2a schematisch stark vereinfacht dargestellt. Zur Vermeidung von Wiederholungen wird lediglich auf wesentliche Unterschiede im Vergleich zu dem vorbeschriebenen Fluidpumpsystem 2 eingegangen. Im Übrigen wird auf die Ausführungen zu dem Fluidpumpsystem 2 nach den Fig. 1 bis 9 verwiesen. Das dort zur grundsätzlichen Funktionsweise und dem grundsätzlichen Aufbau Gesagte gilt mutatis mutandis auch für die Ausführungsform nach Fig. 10.

Das Fluidpumpsystem 2a unterscheidet sich im Wesentlichen dadurch von dem Fluidpumpsystem 2, dass der wenigstens eine dielektrische Elastomeraktor 26a in die Fluidkassette Fa integriert und gemeinsam mit dieser lösbar an dem Aufnahmebereich A des im Übrigen nicht näher dargestellten Gehäuses Ga aufgenommen ist. Folglich weist das Gehäuse Ga keinerlei Aktuatorikkomponenten zur Auslenkung der Pumpmembran 19a auf. Sämtliche hierfür erforderlichen Komponenten sind stattdessen in die Fluidkassette Fa integriert. Dies bietet zahlreiche Vorteile insbesondere im Hinblick auf eine möglichst einfache konstruktive Gestaltung des Gehäuses Ga.

Die Fluidkassette Fa weist wiederum einen formstabilen Grundkörper 18a auf. An diesem ist die Pumpmembran 19a unter Ausbildung wenigstens eines Pumpvolumens 20a festgelegt. Das Pumpvolumen 20a ist in einer dem Pumpvolumen 20 entsprechenden Weise mit einem nicht näher bezeichneten Fluideinlass und Fluidauslass der Fluidkassette Fa fluidleitend verbunden. Den hierfür vorgesehenen Fluidleitungen sind wiederum nicht näher bezeichnete Rückschlagventile zugeordnet.

Bei der anhand Fig. 10 gezeigten Ausführungsform ist der Elastomeraktor 26a ebenfalls als Planaraktor gestaltet und zudem in die Pumpmembran 19a integriert oder umgekehrt. Mit anderen Worten ausgedrückt, bilden die Pumpmembran 19a und der dielektrische Elastomeraktor 26a eine Membrananordnung 19a, 26a. Diese ist unter Ausbildung des Pumpvolumens 20a an dem Grundkörper 18a festgelegt.

Vorliegend weist die Fluidkassette Fa zwei Pumpvolumen 20a, 20'a auf, die auch als erstes Pumpvolumen 20a und zweites Pumpvolumen 20'a bezeichnet werden können. Diese werden mittels der Membrananordnung 19a, 26a fluiddicht voneinander abgetrennt. Je nach Richtung der Stellbewegung S bzw. Auslenkung L der Membrananordnung 19a, 26a wird das erste Pumpvolumen 20a zum Ansaugen von Blut vergrößert und das zweite Pumpvolumen 20'a zum Ausstoßen von Blut verkleinert oder umgekehrt.

Zur Versorgung des dielektrischen Elastomeraktors 26a mit elektrischer Betriebsenergie weist die Fluidkassette Fa elektrische Kontaktelemente 34 auf, die im Aufnahmezustand mit nicht näher gezeigten komplementären Kontaktelementen des Gehäuses Ga elektrisch kontaktiert sind.

Zur Erfassung des Aufnahmezustands der Fluidkassette Fa ist wiederum eine Erfassungseinrichtung 33a vorgesehen. Die Erfassungseinrichtung 33a ist bei der gezeigten Ausführungsform der Fluidkassette Fa zugeordnet, was anhand der strichliert eingezeichneten Wirkverbindung symbolisiert ist. Alternativ kann die Erfassungseinrichtung 33a gehäuseseitig angeordnet und dem Gehäuse Ga zugeordnet sein.

Weiterhin ist eine der Fluidkassette Fa zugeordnete Notbetriebseinrichtung 31a mit einem Antriebselement 32a vorgesehen. Alternativ kann die Notbetriebseinrichtung 31a gehäuseseitig angeordnet und dem Gehäuse Ga zugeordnet sein.

Die Fluidkassette Fa ist als Einwegartikel zur einmaligen Verwendung gestaltet. Entsprechendes gilt für die Fluidkassette F der Ausführungsform nach den Fig. 1 bis 9.

## Patentansprüche

1. Medizinisches Fluidpumpsystem (2a) zur Förderung eines Fluids bei einer extrakorporalen Blutbehandlung, aufweisend
- ein Gehäuse (Ga) mit einem Aufnahmebereich (A),
- eine lösbar an dem Aufnahmebereich (A) aufgenommene Fluidkassette (Fa) mit einem formstabilen Grundkörper (18a) und mit wenigstens einer elastischen Pumpmembran (19a), die unter Ausbildung wenigstens eines Pumpvolumens (20a, 20'a) an dem Grundkörper (18a) festgelegt ist,
- wobei das Pumpvolumen (20a, 20'a) mittels einer Auslenkung (L) der Pumpmembran (19a) zur Förderung des Fluids veränderlich ist,
- und aufweisend wenigstens ein Aktorelement, das zur Auslenkung der Pumpmembran (19a) eingerichtet ist,
- **dadurch gekennzeichnet, dass** das wenigstens eine Aktorelement ein dielektrischer Elastomeraktor (26a) ist, der mit der Pumpmembran (19a) wirkverbunden ist, und der unter Einwirkung einer elektrischen Spannung eine Stellbewegung (S) zur Auslenkung der Pumpmembran (19a) ausführt,
- wobei der wenigstens eine dielektrische Elastomeraktor (26a) in die Fluidkassette (Fa) integriert und gemeinsam mit dieser lösbar an dem Aufnahmebereich (A) aufgenommen ist.

2. Medizinisches Fluidpumpsystem (2a) nach Anspruch 1, **dadurch gekennzeichnet, dass** der wenigstens eine dielektrische Elastomeraktor (26a) als Planaraktor (P) gestaltet ist, wobei der Planaraktor (P) in die Pumpmembran (19a) integriert ist oder umgekehrt.

3. Medizinisches Fluidpumpsystem (2a) nach Anspruch 1 oder 2, **dadurch gekennzeichnet, dass** die Pumpmembran (19a) unter Ausbildung eines ersten Pumpvolumens (20a) und eines zweiten Pumpvolumens (20'a) an dem Grundkörper (18a) festgelegt ist, wobei das erste Pumpvolumen (20a) und das zweite Pumpvolumen (20'a) mittels der Pumpmembran (19a) fluiddicht voneinander getrennt und wechselweise veränderlich sind.

4. Medizinisches Fluidpumpsystem (2a) nach einem der vorhergehenden Ansprüche, **dadurch gekennzeichnet, dass** der wenigstens eine dielektrische Elastomeraktor (26a) zur kapazitiven Erfassung einer Stellposition der Stellbewegung eingerichtet ist und somit zusätzlich als Sensorelement fungiert.

5. Medizinisches Fluidpumpsystem (2a) nach einem der vorhergehenden Ansprüche, **dadurch gekennzeichnet, dass** eine mit der Pumpmembran (19a) wirkverbundene Notantriebseinrichtung (31a) vorgesehen ist, welche ein zur manuell angetriebenen Auslenkung der Pumpmembran (19a) betätigbares Antriebselement (32a) aufweist.

6. Medizinisches Fluidpumpsystem (2a) nach einem der vorhergehenden Ansprüche, **dadurch gekennzeichnet, dass** eine Erfassungseinrichtung (33a) vorgesehen ist, welche zur Erfassung eines Aufnahmezustands der Fluidkassette (Fa) eingerichtet ist, in welchem die Fluidkassette (Fa) bestimmungsgemäß an dem Aufnahmebereich (A) aufgenommen ist.

7. Medizinisches Fluidpumpsystem (2a) nach einem der vorhergehenden Ansprüche, **dadurch gekennzeichnet, dass** der Aufnahmebereich (A) an einer außenliegenden Gehäusewandung (100) des Gehäuses (Ga) angeordnet ist, und dass eine an der Gehäusewandung (100) und/oder dem Grundkörper (18a) ausgebildete Befestigungseinrichtung (B) vorgesehen ist, mittels derer die Fluidkassette (Fa) - in einem an dem Aufnahmebereich (A) aufgenommenen Zustand - an der Gehäusewandung (100) lösbar befestigt ist.

8. Medizinisches Fluidpumpsystem (2a) nach einem der vorhergehenden Ansprüche, **dadurch gekennzeichnet, dass** die Fluidkassette (Fa) als Einwegartikel zur einmaligen Verwendung gestaltet ist.

9. Medizinische Fluidkassette (Fa) für ein medizinisches Fluidpumpsystem (2a) nach einem der vorhergehenden Ansprüche, aufweisend einen formstabilen Grundkörper (18a) und wenigstens eine elastische Pumpmembran (19a), die unter Ausbildung wenigstens eines Pumpvolumens (20a, 20'a) an dem Grundkörper (18a) festgelegt ist, wobei das Pumpvolumen (20a, 20'a) mittels einer Auslenkung der Pumpmembran (19a) zur Förderung des Fluids veränderlich ist, **dadurch gekennzeichnet, dass** wenigstens ein mit der Pumpmembran (19a) wirkverbundener dielektrischer Elastomeraktor (26a) vorgesehen ist, der unter Einwirkung einer elektrischen Spannung eine Stellbewegung (S) zur Auslenkung der Pumpmembran (19a) ausführt, wobei der wenigstens eine dielektrische Elastomeraktor (26a) in die Fluidkassette (Fa) integriert und gemeinsam mit dieser lösbar an einem Aufnahmebereich (A) eines Gehäuses (Ga) des medizinischen Fluidpumpsystem (2a) aufnehmbar ist.

## Claims

1. Medical fluid pump system (2a) for delivering a fluid for extracorporeal blood treatment, comprising
- a housing (Ga) which has a receiving portion (A),
- a fluid cassette (Fa), which is detachably received on the receiving portion (A) and has a dimensionally stable main body (18a) and at least one elastic pump membrane (19a) that is fixed to the main body (18a) with formation of at least one pump volume (20a, 20'a),
- wherein the pump volume (20a, 20'a) is variable by means of a deflection (L) of the pump membrane (19a) for the purpose of delivering the fluid,
- and comprising at least one actuator element configured to deflect the pump membrane (19a),
- **characterized in that** the at least one actuator element is a dielectric elastomer actuator (26a) which is operatively connected to the pump membrane (19a) and, under the influence of an electrical voltage, performs an actuating movement (S) for deflecting the pump membrane (19a),
- wherein the at least one dielectric elastomer actuator (26a) is integrated in the fluid cassette (Fa) and together therewith is detachably received on the receiving portion (A).

2. Medical fluid pump system (2a) according to Claim 1, **characterized in that** the at least one dielectric elastomer actuator (26a) is in the form of a planar actuator (P), wherein the planar actuator (P) is integrated in the pump membrane (19a) or vice versa.

3. Medical fluid pump system (2a) according to Claim 1 or 2, **characterized in that** the pump membrane (19a) is fixed to the main body (18a) with formation of a first pump volume (20a) and a second pump volume (20'a), wherein the first pump volume (20a) and the second pump volume (20'a) are alternately variable and are fluid-tightly separated from one another by means of the pump membrane (19a).

4. Medical fluid pump system (2a) according to one of the preceding claims, **characterized in that** the at least one dielectric elastomer actuator (26a) is configured for capacitive detection of an actuating position of the actuating movement and thus additionally acts as a sensor element.

5. Medical fluid pump system (2a) according to one of the preceding claims, **characterized in that** an emergency drive device (31a), which is operatively connected to the pump membrane (19a) and has a drive element (32a) that can be actuated for manually driven deflection of the pump membrane (19a), is provided.

6. Medical fluid pump system (2a) according to one of the preceding claims, **characterized in that** a detection device (33a), which is configured to detect a receiving state of the fluid cassette (Fa) in which the fluid cassette (Fa) is received as intended on the receiving portion (A), is provided.

7. Medical fluid pump system (2a) according to one of the preceding claims, **characterized in that** the receiving portion (A) is arranged on an external housing wall (100) of the housing (Ga), and **in that** a fastening device (B), which is formed on the housing wall (100) and/or the main body (18a) and by means of which the fluid cassette (Fa) - in a state in which the latter is received on the receiving portion (A) - is fastened detachably to the housing wall (100), is provided.

8. Medical fluid pump system (2a) according to one of the preceding claims, **characterized in that** the fluid cassette (Fa) is in the form of a disposable article for one-time use.

9. Medical fluid cassette (Fa) for a medical fluid pump system (2a) according to one of the preceding claims, comprising a dimensionally stable main body (18a) and at least one elastic pump membrane (19a), which is fixed to the main body (18a) with formation of at least one pump volume (20, 20'a), wherein the pump volume (20a, 20'a) is variable by means of a deflection of the pump membrane (19a) for the purpose of delivering the fluid, **characterized in that** there is provided at least one dielectric elastomer actuator (26a) which is operatively connected to the pump membrane (19a) and, under the influence of an electrical voltage, performs an actuating movement (S) for deflecting the pump membrane (19a), wherein the at least one dielectric elastomer actuator (26a) is integrated in the fluid cassette (Fa) and together therewith can be detachably received on a receiving portion (A) of a housing (Ga) of the medical fluid pump system (2a).

## Revendications

1. Système de pompe à fluide médical (2a) destiné à transporter un fluide lors d'un traitement extracorporel de sang, comportant
- un boîtier (Ga) avec une zone de réception (A),
- une cassette à fluide (Fa) reçue de manière amovible sur la zone de réception (A) avec un corps principal (18a) indéformable et avec au moins une membrane de pompe (19a) élastique, qui est fixée sur le corps principal (18a) tout en formant au moins un volume de pompe (20a, 20'a),
- le volume de pompe (20a, 20'a) pouvant être modifié au moyen d'une déviation (L) de la membrane de pompe (19a) pour le transport du fluide,
- et comportant au moins un élément actionneur, qui est mis au point pour dévier la membrane de pompe (19a),
- **caractérisé en ce que** l'au moins un élément actionneur est un actionneur élastomère diélectrique (26a), qui est en liaison active avec la membrane de pompe (19a) et qui, sous l'action d'une tension électrique, exécute un mouvement d'actionnement (S) pour dévier la membrane de pompe (19a),
- l'au moins un actionneur élastomère diélectrique (26a) étant intégré dans la cassette à fluide (Fa) et étant reçu conjointement avec celle-ci de manière amovible sur la zone de réception (A).

2. Système de pompe à fluide médical (2a) selon la revendication 1, **caractérisé en ce que** l'au moins un actionneur élastomère diélectrique (26a) est réalisé sous la forme d'un actionneur plan (P), l'actionneur plan (P) étant intégré dans la membrane de pompe (19a) ou inversement.

3. Système de pompe à fluide médical (2a) selon la revendication 1 ou 2, **caractérisé en ce que** la membrane de pompe (19a) est fixée sur le corps principal (18a) tout en formant un premier volume de pompe (20a) et un deuxième volume de pompe (20'a), le premier volume de pompe (20a) et le deuxième volume de pompe (20'a) étant séparés l'un de l'autre de manière étanche aux fluides au moyen de la membrane de pompe (19a) et pouvant être modifiés tour à tour.

4. Système de pompe à fluide médical (2a) selon l'une des revendications précédentes, **caractérisé en ce que** l'au moins un actionneur élastomère diélectrique (26a) est mis au point pour détecter de manière capacitive une position de réglage du mouvement de réglage et fonctionne ainsi en supplément comme un élément de détection.

5. Système de pompe à fluide médical (2a) selon l'une des revendications précédentes, **caractérisé en ce qu'**est prévu un dispositif d'entraînement de secours (31a) en liaison active avec la membrane de pompe (19a), lequel comporte un élément d'entraînement (32a) pouvant être actionné pour la déviation à entraînement manuel de la membrane de pompe (19a).

6. Système de pompe à fluide médical (2a) selon l'une des revendications précédentes, **caractérisé en ce qu'**est prévu un dispositif de détection (33a), qui est mis au point pour détecter un état de réception de la cassette à fluide (Fa), dans lequel la cassette à fluide (Fa) est reçue conformément à l'usage prévu sur la zone de réception (A).

7. Système de pompe à fluide médical (2a) selon l'une des revendications précédentes, **caractérisé en ce que** la zone de réception (A) est disposée sur une paroi de boîtier extérieure (100) du boîtier (Ga), et qu'est prévu un dispositif de fixation (B) formé sur la paroi de boîtier (100) et/ou sur le corps principal (18a), au moyen duquel la cassette à fluide (Fa) est fixée de manière amovible sur la paroi de boîtier (100), dans un état reçu sur la zone de réception (A).

8. Système de pompe à fluide médical (2a) selon l'une des revendications précédentes, **caractérisé en ce que** la cassette à fluide (Fa) est conçue comme un article jetable à usage unique.

9. Cassette à fluide médicale (Fa) pour un système de pompe à fluide médical (2a) selon l'une des revendications précédentes, comportant un corps principal (18a) indéformable et au moins une membrane de pompe (19a) élastique, qui est fixée sur le corps principal (18a) tout en formant au moins un volume de pompe (20a, 20'a), le volume de pompe (20a, 20'a) pouvant être modifié au moyen d'une déviation de la membrane de pompe (19a) pour le transport du fluide, **caractérisée en ce qu'**est prévu au moins un actionneur élastomère diélectrique (26a) en liaison active avec la membrane de pompe (19a), qui exécute, sous l'action d'une tension électrique, un mouvement de réglage (S) pour la déviation de la membrane de pompe (19a), l'au moins un actionneur élastomère diélectrique (26a) étant intégré dans la cassette à fluide (Fa) et pouvant être reçu conjointement avec celle-ci de manière amovible sur une zone de réception (A) d'un boîtier (Ga) du système de pompe à fluide médical (2a).
